# EUROPEAN PATENT APPLICATION

(11) **EP 2 415 413 A1**
(43) Date of publication of application: **08.02.2012**
(21) Application number: 10171822.9
(22) Date of filing: 04.08.2010
(51) Int. Cl.: A61B 17/80, A61L 24/00

(54) **Device for enhancement of bone fixation**

(71) Applicant: Ozics Oy, 33230 Tampere (FI)
(72) Inventor: Pösel, Andreas, STJ04, Swieqi (MT); Kaikkonen, Auvo, SLM1601, Sliema (MT)
(74) Representative: Kaukonen, Juha Veikko

(57) **Abstract**

An arrangement for bone support. The arrangement comprises a support device comprising a device body having a bottom surface (3). The arrangement further comprises a filling agent (2) possessing ability to convert from a flowable state into a non-flowable state, said filling agent (2) being provided in said flowable state under said bottom surface (3) of the support device. The filling agent (2) is arranged to convert from said flowable state into said non-flowable state in contact with the bottom surface (3) of the support device.

## Description

### FIELD OF THE INVENTION

The present invention relates to an arrangement for bone support.

### BACKGROUND OF THE INVENTION

When a bone fractures, it is immobilized by external or internal fixation in order to give the bone opportunity to build new callus and heal so it can again carry the intended loads.

External fixation is accomplished by the use of a plaster cast, or in more complex scenarios, by use of external fixation devices, e.g. scaffolds outside the body that keep the bone in the right position during healing.

Internal fixation is typically achieved by means of screws, plates, intramedullary nails, wires, cables, or combination thereof, herein referred to as fracture fixation devices.

The fracture fixation devices are applied on the bone surface following the principles of open reduction and internal fixation (ORlF). The fracture fixation devices are used especially in cases of unstable fractures or if it is anticipated that external immobilization with a plaster cast or alike alone is not sufficient. This is the case for instance in some of the fractures of the facial skeleton and upper or lower extremities such as hip fractures, mandibular fractures or distal radius fractures.

The repositioned and immobilized bone then is given opportunity to heal and gradually take over the loads which result from daily activities.

In principle, osteosynthesis with metallic plates is an effective method to allow for bone regeneration and callus formation. These plates are typically made from stainless steel or titanium, i.e. materials that are very stiff and strong enough to facilitate a sufficient immobilization.

The plate shall conform to the bone topology, i.e. to the shape of the bone surface, as good as possible to render sufficient fixation strength. The bone topology is, however, different in every patient and that is the reason why the plates cannot be preformed to the bone topology already in their production process. The form of the plate leaving its production process may only be an approximate of the patient's bone topology. In other words, the plate is, more or less, a semi-finished product that shall be formed to adapt to the surface of the specific bone tissue to be operated.

The plate may be formed with surgical pliers to fit to the bone surface. However, this kind of forming is particularly difficult with the plates made of stiff materials, in particular with metallic plates, to form the plate to adapt with the bone topology. Usually the adaption does only succeed roughly and varies in quality from one operator to another.

If the plate does not perfectly conform to the bone, it will contact the bone in a few points only. The contact points potentially create local stress peaks that require special attention in the plate design resulting typically as unnecessarily thick plates to be applied. This situation is not significantly improved even when screws are placed through the plates into the bone. The placement and tightening of the screws will also create pressure points between the plate and the bone. These stress concentrations may lead to bone necrosis, an irreversible damage of the bone that may trigger further unwanted physiological reactions. Said contact points create also concentration of high loads where the bone tissue may yield or even collapse. This effect may lead to inferior fixation strengths of the whole construct. As a further consequence, screws in such an imperfect construct may be exposed to bending stresses or shear forces due to the unpredictable movements of the plate on the contact points when loaded externally. This may break or pull out the screw prematurely.

### BRIEF DESCRIPTION OF THE INVENTION

An object of the present invention is thus to provide an arrangement for bone support to overcome the above problems. The objects of the invention are achieved by an arrangement which is characterized by what is stated in the independent claim. The preferred embodiments of the invention are disclosed in the dependent claims.

The invention is based on the idea of immobilizing a fractured bone by use of an arrangement which comprises a support device such as an osteosynthesis plate and filling agent that is arranged between the support device and the bone tissue. The filling agent is applied in the bottom surface of the support device and/or on the bone surface in a flowable state, i.e. in form of flowable mass or paste. The filling agent hardens or sets or cures then into a form of load bearing mass that fills the gaps and cavities between bone and the support device. The filling agent preferably adheres to the support device, bone tissue or both by chemical bonds and/or physical adhesion.

An advantage of the invention is that stresses are evenly distributed between bone and the bottom surface of the support device, preferably throughout the complete bottom surface of the support device facing the bone. Thus stress concentrations and the resulting potential drawbacks described above can be avoided. Also all the stiffness and strength of the support device may then contribute to the overall stiffness and strength of the arrangement. Thanks to this adequate fixation strength may be achieved with a smaller, i.e. thinner or lighter, support device or with using a smaller number of screws than in prior art constructs.

According to an embodiment of the invention the filling agent adheres chemically to the support device and/or to bony tissue. According to another embodiment of the invention the filling agent adheres by physical bonds, e.g. form fit, to the support device and/or to bony tissue. An advantage is that even smaller support devices may be used and still a sufficient stiffness and strength of the bone support are achieved.

According to still another embodiment of the invention the device and the filling agent attached thereon establish a layer structure wherein the strength of the device alone is not sufficient to immobilize the fractured bone. An advantage is that the support device may be construed so small and thin that it is usable in areas where soft tissue is limited and the skin covering the fractured bone may already be strained.

According to still another embodiment of the invention the bottom surface of the device comprises a concave geometry for receiving the filling agent. An advantage is that better mechanical and/or chemical fixation between the device and the filling agent is achieved.

### BRIEF DESCRIPTION OF THE DRAWINGS

In the following the invention will be described in greater detail by means of preferred embodiments with reference to the accompanying drawings, in which
Figure 1a is a schematic view of a cross section of an embodiment of the invention comprising an osteosynthesis plate attached to bony tissue by fixation screws and filling agent,
Figure 1b is a schematic view of a longitudinal cross section of the embodiment shown in Figure 1 a,
Figure 2 is a schematic view of a longitudinal cross section of a second embodiment of the invention comprising an osteosynthesis plate attached to bony tissue solely by filling agent,
Figure 3 is a schematic view of a cross section of a support device of an embodiment of the invention, and
Figure 4 is a schematic view of a cross section of a second support device of an embodiment of the invention.

For the sake of clarity, the figures show the invention in a simplified manner. Like reference numbers identify like elements.

### DETAILED DESCRIPTION OF THE INVENTION

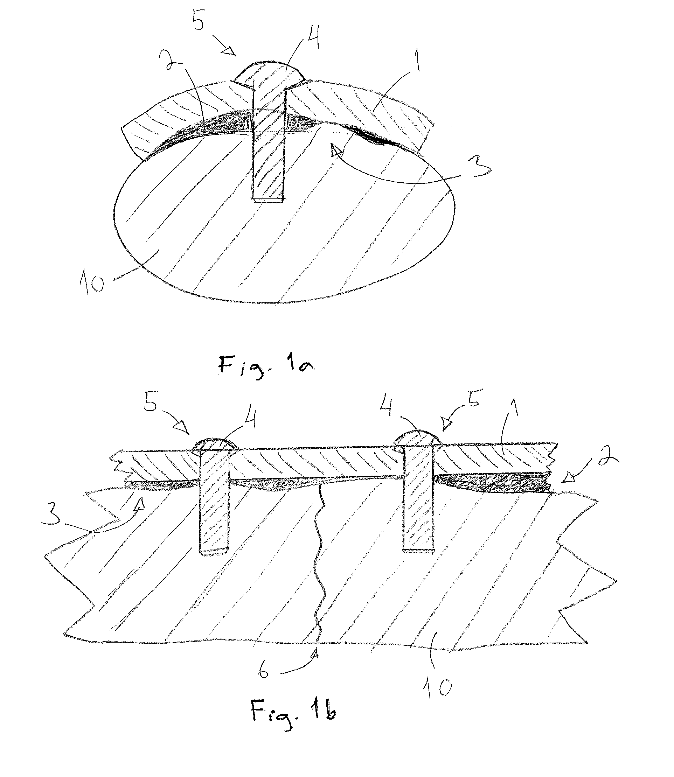

Figures 1 a and 1 b are a schematic views of an embodiment of the invention comprising an osteosynthesis plate, fixation screws and filling agent.

Preferably, the plate 1 has been shaped mechanically to the topology of the bone 10.

In addition to the mechanical adaptation of the plate 1, filling agent 2 is applied between the plate and the bone 10.

The filling agent 2 is applied on the bottom surface 3 of the plate 1. The "bottom surface" means the surface which is arranged towards the bone to which the plate 1 is attached.

The filling agent 2 is in form of fluid or paste-like during its applying. The viscosity of the filling agent 2 is preferably adjusted so that it is quite easily spread to either the bottom surface 3 and/or the bone surface 10 but on the other hand it does not run or leak away from said surfaces.

The filling material 2 may be poured, injected, smoothed, brushed etc. on its place. In an embodiment of the invention the plate 1 is arranged to its correct place on the bone 10 and attached therein by fixation means, such as screws, after which the filling material 2 is injected in the free space between the plate 1 and the bone 10.

The plate 1 is then pressed onto the bone 10 until all excess filling agent 2 is squeezed out from the sides of the plate and the excess is removed where necessary before the filling agent 2 is cured. The viscosity of the filling agent 2 and the way of its application is such that a facture gap 6 between bone parts will not be populated by the filling agent 2, at least not to a great extent.

The filling agent 2 is selected so that it adheres chemically, i.e. by chemical bonds, or by physical bonds, or by both to the plate 2 or the bone 10. More preferably, the filling agent 2 is selected so that it adheres both to the plate 1 and to the bone 10.

The arrangement according to the invention may comprise fixation means such as one or more screws 4, and the plate 1 corresponding fixation holes 5. The screw 4 may be arranged in the fixation hole 5 before of after curing of the filling agent 2.

Among other fixation means suitable for use in the arrangement according to the invention are pins, nails, staples etc.

The filling agent 2 adheres preferably both to the bone 10 and the plate 1. This provides additional strength and rigidity in the bone-plate interface. In addition to this advantage of reinforcement the filling agent 2 may help to position and hold the plate 1 in the right position on the bone 10 before the screws 4 are placed through the fixation holes 5.

The filling agent 2 may be resorbable or non-resorbable material. As used herein, the term "resorbable" means that the material is biodegradable, bioerodible or bioabsorbable. By "biodegradable" it is meant that the composition will degrade over time by the action of enzymes, by hydrolyric action and/or by other similar mechanisms in the human body. By "bioerodible," it is meant that the composition will erode or degrade over time due, at least in part, to contact with substances found in the surrounding tissue fluids, cellular action, and the like. By "bioabsorbable," it is meant that the composition will be broken down and absorbed within the human body, for example, by a cell, a tissue, and the like.

The filling agent 2 may comprise a polymeric matrix. Alternatively, the filling agent 2 may comprise a ceramic matrix or a matrix that is mixture of polymer and ceramic. The polymeric matrix is preferably biostabile or non-resorbable. Some examples of matrix materials are listed here: methyl-acrylates, such as but not limited to poly(methyl meth-acrylate) (PMMA), tetra-ethyleneglycol dimethacrylate (TEGDMA), hydroxyethyl methacrylate (HEMA), bisphenolglycidyl dimethacrylate (BISGMA), urethane dimethacrylate (UDMA), and cyanoacrylates; polyurethanes, polylactic acid (PLA), polyglycolic acid (PGA), polyanhydrides, glass ionomer composites, calcium-phosphates, calcium-sulphates or combinations thereof.

The filling agent 2 may also contain one or more filler materials mixed with the polymeric matrix. The filler material may comprise fillers such as resorbable or non-resobable glasses, calcium phosphates, calcium sulfates, polymeric fillers, metallic fillers, tricalciumphosphate (TCP), nano-TCP, magnesium particles or other metallic particles or powder, glass fibers, silicone carbide, carbon fibers, polymer spheres such as PMMA beads, polymer fibers, radiopaque fillers, such as BaSO₄, titanium oxide or composition thereof.

The filling agent 2 can be light curable or chemically curable. The filling agent 2 may contain plasticizers to enhance the flexibility in order to avoid failure under load.

The curing time of the filling agent 2 is preferably between 1 and 30 minutes after its application. The filling agent 2 may be machined after its curing.

The size of the plate 1 and/or the size or number of the fixation means may be reduced compared to prior art because equal fixation strength may be achieved because the contact area between the plate 1 and the bone 10 is maximized and stress concentrations are avoided. This is a significant advantage for the patient because smaller incisions and surgical exposures of the fractures are sufficient to introduce the arrangement in the surgical site on the bone 10. Thus the trauma for the patient is minimized. This is particularly advantageous in areas where subcutaneous tissue is thin or limited and the skin covering the fractured bone is already stretched because of the swelling caused by the trauma or the surgery itself, such as in an ankle or a forearm. Furthermore, it is to be noted that metallic plate often stays within the body and is not surgically removed because a removal operation would cause expenses and expose the patient to another anesthesia and surgical operation. Thus it is advantageous to minimize the mass of metallic plate or other type of support device that will reside permanently in the patient's body. A minimization of the mass also results in lower profiles of the support device and decreases palpability and visibility of the fixation arrangement. According to an embodiment of the invention the support device alone is not sufficient to immobilize the fractured bone. In this embodiment the filling agent attached to the support device establishes a composite structure together with said support device the strength of which is sufficient to immobilize the bone for regenerating the bone.

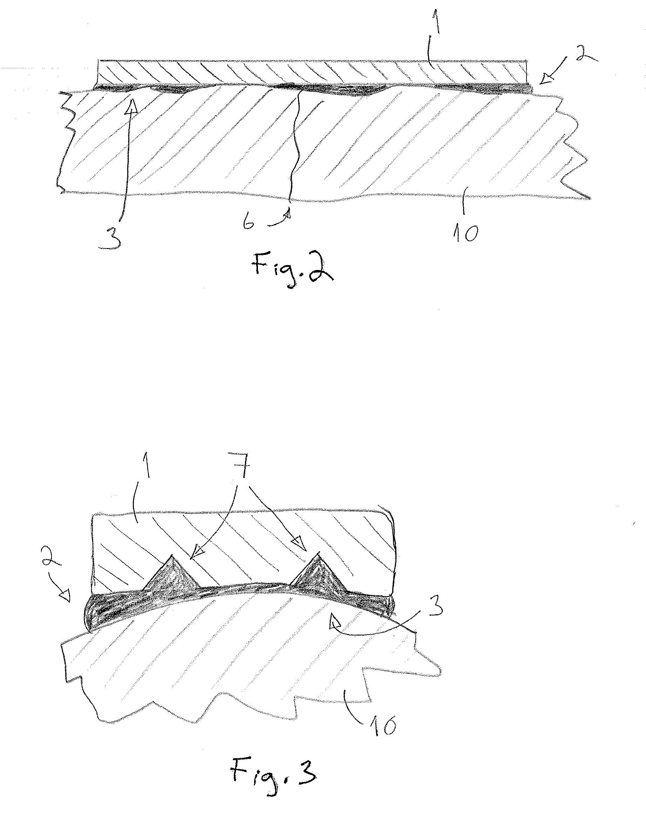

Figure 2 is a schematic view of an embodiment of the invention comprising an osteosynthesis plate and filling agent. The main difference of this embodiment compared to the previous one is that it does not comprise fixation means, e.g. screws, at all. Instead, the plate 1 is adhered to the bone 10 only by the filling agent 2. This kind of arrangement is especially suitable for operations where the fractured bone 10 is a non-load bearing bone or a low-load bearing bone. This way the use of fixation means, e.g. screws, rivets or pins, may be avoided, which leads to a shorter operation time and, furthermore, trauma for the patient is minimized.

The filling agent 2 is selected so that it adheres chemically and/or physically, i.e. by chemical and/or physical bonds to the plate 1 and the bone 10. The filling agent 2 may comprise one or more already mentioned materials.

It is to be noted here that the plate 1 or any other type of support device may be manufactured from a biocompatible grade metal, metal alloy, plastic or plastic composite, or ceramics. Some examples of the materials are stainless steel, magnesium, titanium, Nitinol, tantalum, niobium, carbon-fiber, silicone-carbide, fiberglass, bioglass, Kevlar and PEEK (polyether-etherketone).

According to another embodiment of the invention, the support device 1 comprises resorbable material.

Figure 3 is a schematic view of a cross section of a support device of an embodiment of the invention.

The bottom surface 3 of the support device, here a plate 1, comprises a geometry that increases the contact area of the plate 1 and the filling agent 2 that will be arranged in contact with the bottom surface 3. The geometry may comprise concave shapes such as one or more grooves 7, point-form cavities, recesses etc. The cross-section of the concave geometry may, of course, differ from one shown in Figure 3.

The concave geometry receives some amount of the filling agent 2. The filling agent 2 hardening in the concave geometry contributes to the mechanical attachment between the plate 1 and the filling agent.

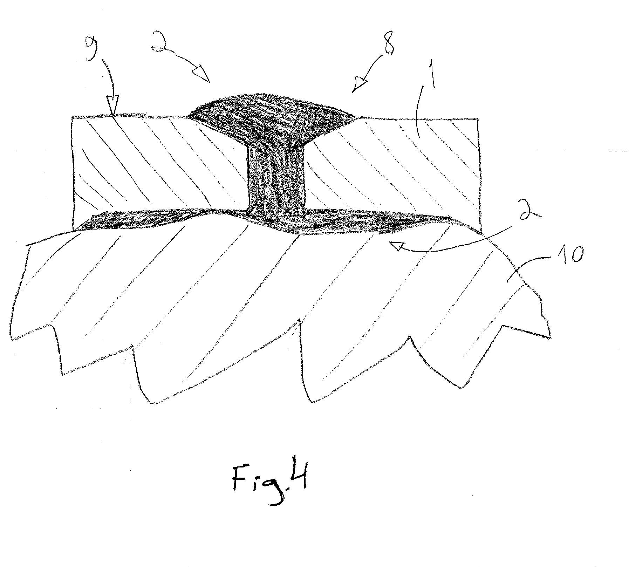

Figure 4 is a schematic view of a cross section of support device of an embodiment of the invention.

The support device is here again a plate 1 having a bottom surface 3. The bottom surface 3 comprises a hole 8 that extends through the plate to an upper surface 9 thereof. A filling agent 2 is arranged between the bottom surface 3 of the plate and a bone 10. The filling agent 2 fills not only the space between the plate 1 and the bone 10 but also the hole 8. A portion of the filling agent 2 has occupied part of the upper surface 9 of the plate 1. The upper surface 9 may comprise a countersink for receiving the filling agent 2. The plate is locked on the bone as a consequence of hardening of the filling agent 2.

The filling agent 2 may be injected between the plate 1 and the bone 10 through the one or more holes 8.

It will be obvious to a person skilled in the art that, as the technology advances, the inventive concept can be implemented in various ways. The invention and its embodiments are not limited to the examples described above but may vary within the scope of the claims.

## Claims

1. An arrangement for immobilizing a fractured bone (10), comprising a support device comprising a device body having a bottom surface (3), **characterized in that**
the arrangement further comprises a filling agent (2) possessing ability to convert from a flowable state into a non-flowable state,
said filling agent (2) being provided in said flowable state under said bottom surface (3) of the support device,
- the filling agent (2) being arranged to convert from said flowable state into said non-flowable state in contact with the bottom surface (3) of the support device.

2. The arrangement as claimed in claim 1, **characterized in that** the support device is an osteosynthesis plate (1).

3. The arrangement as claimed in claim 1 or 2, **characterized in that** the support device comprises at least one hole (5, 8) extending through the device body, and that a portion of the filling agent (2) being arranged in said hole (5, 8).

4. The arrangement as claimed in any of the preceding claims, **characterized in that** the support device comprises non-resorbable material.

5. The arrangement as claimed in any of the preceding claims, **characterized in that** the filling agent (2) comprises one or more materials selected from acrylic polymers, such as methacrylates, BISGMA, HEMA, TEGDMA, UDMA, glass ionomer composites, light curable PLAs, cyanoacrylates, or curable ceramic compositions such as calcium-sulphate or calcium-phosphate.

6. The arrangement as claimed in any of the preceding claims, **characterized in that** the filling agent (2) comprises one or more fillers selected from TCP (tricalciumphosphate), calcium-sulphate, magnesium particles and/or powder, glass fibers, polymer spheres, polymer fibers, radiopaque fillers.

7. The arrangement as claimed in any of the preceding claims, **characterized in that** the filling agent (2) is arranged to adhere chemically to the support device.

8. The arrangement as claimed in any of the preceding claims, **characterized in that** the filling agent (2) is arranged to adhere chemically to bony tissue (10).

9. The arrangement as claimed in any of the preceding claims, **characterized in that** the filling agent (2) is arranged to adhere physically to the support device.

10. The arrangement as claimed in any of the preceding claims, **characterized in that** the filling agent (2) is arranged to adhere physically to bony tissue (10).

11. The arrangement as claimed in any of the preceding claims, **characterized in that** the bottom surface (3) of the device comprises a concave geometry for receiving the filling agent (2).

12. The arrangement as claimed in any of the preceding claims, **characterized in that** the device and the filling agent (2) attached thereon are arranged to establish a composite structure wherein the strength of the device alone is not sufficient to immobilize the fractured bone (10).
